# EUROPEAN PATENT APPLICATION

(11) **EP 4 796 058 A1**
(43) Date of publication of application: **26.08.2026**
(21) Application number: 25159256.4
(22) Date of filing: 21.02.2025
(51) Int. Cl.: A61B 17/3207

(54) **LESION MACERATON CATHETERS**

(71) Applicant: Koninklijke Philips N.V., 5656 AE Eindhoven (NL)
(72) Inventor: MACCONNELL, Michael Bryan, Eindhoven (NL); VAN DER HORST, Arjen, Eindhoven (NL); MILAITIS, Maxwell, Eindhoven (NL)
(74) Representative: Philips Intellectual Property & Standards

(57) **Abstract**

A lesion maceration catheter includes an elongate catheter body, a cutting head assembly and an eccentric positioning element. The cutting head assembly is disposed at a distal end of the catheter body. The cutting head assembly includes a protective cage with an opening in a side surface of the protective cage, and a cutting element within the protective cage and exposed through the opening. The eccentric positioning element is configured to press the opening of the cutting head assembly against a vessel wall.

## Description

### FIELD OF INVENTION

The inventive concepts generally relate to medical devices and methods for treating vascular conditions, and more particularly, to a catheter-based system for removing collagenous lesions from vessels using a vessel wall facing cutting mechanism with positioning elements. The invention relates to a lesion maceration catheter and corresponding method.

### BACKGROUND

Venous thrombotic lesions present a significant challenge in vascular medicine, particularly when they occur in deep veins such as the profunda femoris vein. These lesions, often rich in collagen, can impede blood flow and contribute to various complications, including decreased inflow into the common femoral vein and compromised stent patency. The presence of such lesions increases the risk of recurrent thrombosis and can significantly impact a patient's quality of life.

Current treatment approaches for venous thrombotic lesions suffer several limitations. Traditional catheter-based methods and surgical interventions often struggle to effectively target and remove collagen-rich lesions, especially those located in deep veins. High-pressure balloon venoplasty, while commonly used, raises concerns about potential vessel damage and re-thrombosis. Mechanical thrombectomy techniques have shown limited efficacy in addressing wall-adherent collagen deposits.

The management of chronic collagenous lesions within the deep venous system represents an area of unmet clinical need. Existing devices and treatment modalities often lack the precision and versatility required to safely and effectively remove these challenging lesions. The inefficiency of current approaches in managing post-thrombotic lesions, particularly collagen-rich lesions in the profunda femoris vein, underscores the need for advanced, minimally invasive technologies.

Furthermore, the diverse morphological and spatial variations within the venous system pose additional challenges for existing treatment devices. Many current tools lack the necessary maneuverability and precision to access and treat lesions effectively across different vascular anatomies. This limitation can result in suboptimal treatment outcomes and may necessitate multiple interventions.

### SUMMARY

According to an aspect of the inventive concepts, a lesion maceration catheter is provided that includes an elongate catheter body, a cutting head assembly and an eccentric positioning element. The cutting head assembly is disposed at a distal end of the catheter body. The cutting head assembly includes a protective cage with an opening in a side surface of the protective cage, and a cutting element within the protective cage that is exposed through the opening. The eccentric positioning element is configured to press the opening of the cutting head assembly against a lesion on a vessel wall, thereby pushing the lesion into the opening in the cutting head assembly.

The eccentric positioning element may be positioned along an exterior of the elongate catheter body. The eccentric positioning element may be an inflatable balloon.

The cutting element may include a cutting blade. The cutting blade in cooperation with an edge of the opening in the protective cage may be configured to macerate lesions of the vessel wall. The cutting blade and/or the edge of the opening in the protective cage may be serrated. The cutting blade may be a rotating cutting blade or a translating cutting blade.

The elongate catheter body may include a material transport lumen for removal of lesion pieces macerated by the cutting head assembly, and the material transport lumen may be in fluid communication with an interior of the cutting head assembly.

The lesion maceration catheter may further include a rotating cutting blade within the catheter that is configured to further cut the lesion pieces macerated by the cutting head assembly.

According to another aspect of the inventive concepts, a lesion maceration catheter is provided that includes an elongate catheter body, a cutting head assembly, an inflatable balloon, an inflation lumen and a material transport lumen. The cutting head assembly disposed at a distal end of the catheter body. The cutting head assembly includes a protective cage with an opening in a side surface of the protective cage, and a rotational or translating cutting blade within the protective cage and exposed through the opening. The inflatable balloon is mounted to the elongate catheter body, and is configured when inflated to press the opening of the protective cage against a lesion on a vessel wall, thereby pushing the lesion into the opening in the cutting head assembly. The inflation lumen is located within the elongate catheter body in fluid communication with the inflatable balloon. The material transport lumen is located within the elongate catheter body in fluid communication with an interior of the cutting head assembly.

The cutting head assembly may be independently movable in a longitudinal direction relative to the catheter body, and/or independently rotatable about a longitudinal axis relative to the catheter body.

The lesion maceration catheter may further include an intravascular imaging device at the distal end of the catheter body.

The lesion maceration catheter may further include a pump at a proximal end of the elongate catheter body in fluid communication with the inflation lumen, a pump at the proximal end of the elongate catheter in fluid communication with the material transport lumen, and a drive motor at the proximal end of the elongate catheter operatively connected to the rotational or translating cutting blade by at least one drive shaft extending through the catheter body.

According to still another aspect of the inventive concepts, a method for macerating a vasculature lesion is provided that includes advancing a catheter into a vessel, the catheter including a cutting head assembly with a cutting element facing outwardly towards a vessel wall from a longitudinal axis of the catheter. The method further includes positioning the cutting head assembly adjacent to a lesion of the vessel wall, activating an eccentric positioning element to press the cutting element against the lesion, and actuating the cutting element to macerate the lesion.

### BRIEF DESCRIPTION OF THE DRAWINGS

The above and other aspects and features of the inventive concepts will become readily apparent from the detailed description that follows, with reference to the accompanying drawings, in which:
FIG. 1 is a system level block diagram for reference in describing a catheter system according to an example embodiment of the inventive concepts;
FIG. 2 is perspective view of a lesion maceration catheter system according to an example embodiment of the inventive concepts;
FIGS. 3A through 3C are schematic and cut-away views of a cutting head assembly according to some embodiments of the inventive concepts;
FIGS. 4 through 8 are perspective views of lesion maceration catheters according to employing mechanical cutters according to example embodiments of the inventive concepts;
FIG. 9 is perspective view of a lesion maceration catheter employing an RF cutter according to an example embodiment of the inventive concepts;
FIG. 10 is perspective view of a lesion maceration catheter employing two eccentric positioning elements according to an example embodiment of the inventive concepts;
FIG. 11 is perspective view of a lesion maceration catheter employing a laser cutter according to an example embodiment of the inventive concepts;
FIG. 12 is perspective view of a lesion maceration catheter employing nitinol arc as an eccentric positioning element according to an example embodiment of the inventive concepts;
FIG. 13 and 14 are perspective views of a blade extension cam mechanism according to an example embodiment of the inventive concepts;
FIG. 15 is perspective view of a lesion maceration catheter employing pull wires as an eccentric positioning element according to an example embodiment of the inventive concepts;
FIGS. 16 and 17 are perspective view of a lesion maceration catheter system incorporating intravascular imaging according to example embodiments of the inventive concepts;
FIG. 18 is perspective view of a lesion maceration catheter including an imaging array mounted in a cutter head assembly according to an example embodiment of the inventive concepts; and
FIG. 19 is a flowchart for reference in describing a method of macerating vasculature lesions according to an embodiment of the inventive concepts.

### DETAILED DESCRIPTION

In the following detailed description, for purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the present disclosure.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

Relative terms, such as "above," "below," "top," "bottom," "upper" and "lower" may be used to describe the various elements' relationships to one another, as illustrated in the accompanying drawings. These relative terms are intended to encompass different orientations of the device and/or elements in addition to the orientation depicted in the drawings. For example, if the device were inverted with respect to the view in the drawings, an element described as "above" another element, for example, would now be "below" that element. Similarly, if the device were rotated by 90° with respect to the view in the drawings, an element described "above" or "below" another element would now be "adjacent" to the other element; where "adjacent" means either abutting the other element, or having one or more layers, materials, structures, etc., between the elements.

The drawings may in some cases focus on structural features of embodiments of the inventive concepts. However, the drawings may not be drawn to scale, and relative dimensions of different structural elements may differ from those depicted in the drawings. Further, throughout the drawings, like reference numbers refer to the same or similar elements.

As the field of vascular medicine continues to advance, there is a growing recognition of the need for innovative solutions that can address the complexities of venous thrombotic lesions. Improved technologies that offer precise, comprehensive, and safe lesion removal while maintaining the integrity of the surrounding vascular structures are highly desirable. Such advancements could potentially enhance treatment efficacy, reduce procedural complications, and improve long-term outcomes for patients suffering from deep vein thrombosis and related conditions.

In at least some embodiments of the inventive concepts, the introduction of a morcellator-inspired cutting mechanism represents an advancement in the treatment of chronic collagenous lesions within the deep venous system. Leveraging the proven success of morcellators in laparoscopically removing uterine fibroids, innovative devices described herein employ the cutting force generated by morcellators to effectively address and remove collagen-rich fibrotic lesions within the vascular system. By incorporating a steerable catheter body and a motor-driven rotational or translational cutting head, the devices offer a controlled and directed approach to macerate the lesion, providing targeted treatment to the specific lesion site. Safety concerns, particularly regarding the potential for vessel perforation, are recognized as critical design considerations, and the devices addresses these concerns by prioritizing a side-facing cutting mechanism over a front-facing one to minimize the risk of perforation and other safety-related issues. Additionally, the incorporation of an eccentric positioning element such as an eccentric balloon or an adjustable nitinol arc, mounted 180 degrees opposite of the cutting head, aims to ensure that the cutting head can be pressed into the lesion, minimizing the potential for guttering, increasing the amount of lesion that is forced into the cutting mouth, and achieving more precise and controlled lesion removal. The versatility and adaptability of the devices are also central to its efficacy, as the cutting-head-balloon subassembly may be engineered to rotate 360 degrees, facilitating access to any location within the vessel and enabling comprehensive and thorough treatment of the lesion. The mechanism of action of the cutting head, encompassing various solutions such as a rotating cutting blade, a translating cutting blade, or an RF cutting blade, further underscores the potential of the device to offer tailored and versatile treatment options for chronic collagenous lesions within the deep venous system.

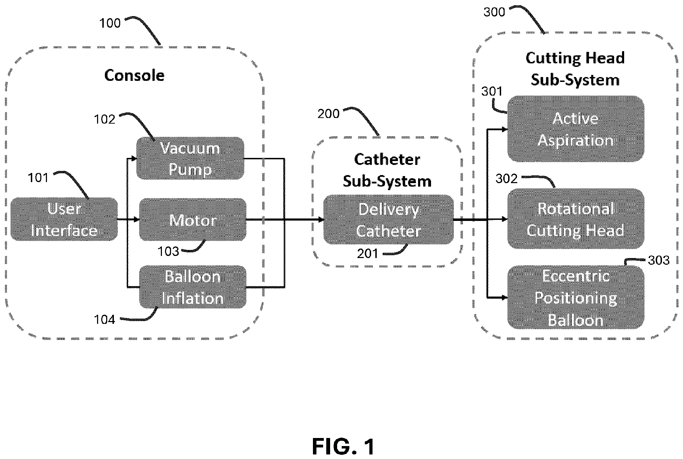

FIG. 1 is a system level block diagram for reference in describing a catheter system according to an example embodiment of the inventive concepts.

Referring to FIG. 1, the depicted system includes a console 100, a catheter sub-system 200, and a cutting head sub-system 300. The console 100 may include a user interface 101, a motor 103, a vacuum pump 102, and a balloon inflation pump 104. In example embodiments, the user interface 101 allows an operator to control the system of FIG. 1, including the motor 103, vacuum pump 102, and balloon inflation pump 104. In alternative embodiments, one or more of the motor 103, vacuum pump 102 and balloon inflation pump 104 are controlled individually, i.e., without using a user interface such as the user interface 101.

The motor 103 may be configured to drive a cutting element of the catheter-based system. In some example embodiments, the motor 103 is electrically powered and is capable of providing rotational motion to the cutting element. For example, the motor 103 may be connected to the cutting head sub-system 300 via a rotational drive shaft (not shown) of the catheter sub-system 200, transmitting mechanical power to the cutting element for lesion maceration.

The vacuum pump 102 may be configured to provide aspiration through the catheter-based system of FIG. 1. In example embodiments, the vacuum pump 102 generates negative pressure, which may be transmitted through a material transport lumen in the catheter sub-system 200 to the cutting head sub-system 300. This negative pressure may facilitate the removal of macerated lesion pieces from the treatment site.

The balloon inflation pump 104 may be configured to control an eccentric positioning balloon of the catheter-based system. In example embodiments, the balloon inflation pump104 inflates and deflates an eccentric positioning balloon (described later) to adjust its radial force against a vessel wall. The balloon inflation pump 104 may be connected to the cutting head sub-system 300 through an inflation lumen in the catheter sub-system 200, providing a mechanism for inflating and deflating the eccentric positioning balloon. In example embodiments, the balloon inflation pump 104 is operable to inflate the eccentric positioning balloon with a saline or a mix of saline and contrast agent.

The catheter sub-system 200 includes a delivery catheter 201, which serves as a conduit between the console 100 and the cutting head sub-system 300. The delivery catheter 201 facilitate positioning of the cutting head sub-system within a vessel, transmission of mechanical power from the motor 103 to the cutting head sub-system 300, aspiration from the vacuum pump 102 to the cutting head sub-system 300, and balloon inflation/deflation from the balloon inflation pump 104 to the cutting head sub-system 300.

The cutting head sub-system 300 of the example of FIG. 1 includes an active aspiration mechanism 301, a rotational cutting head 302, and an eccentric positioning balloon 303.

The active aspiration mechanism 301 may be space or cavity defined within the rotational cutting head 302 that is in fluid communication with the vacuum pump 102 via the delivery catheter 201.

The rotational cutting head 302 is driven by the motor 103 as explained above, providing a mechanism for lesion maceration.

The eccentric positioning balloon 303 is inflated and deflated by the balloon inflation pump 104 as explained above, enabling precise positioning of the cutting head 302 within the vessel. This aspect of the inventive concepts will be described in greater detail below in the context of exemplary embodiments.

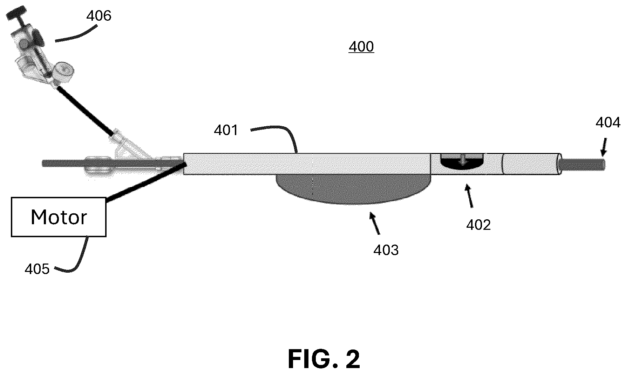

FIG. 2 is perspective view of a lesion maceration catheter system according to an example embodiment of the inventive concepts.

Referring to FIG. 2, the system 400 includes an elongate catheter body 401 and a cutting head assembly 402 disposed at a distal end of the catheter body 401. As will be explained later, the cutting head assembly 402 includes a protective cage with an opening in a side surface of the protective cage, and a cutting element within the protective cage that is exposed through the opening. In FIG. 2, the arrow of the cutting head assembly represents the direction in which the cutting elements traverses in within the protective cage. That is, in the example of FIG. 2, the cutting element is a rotational cutting element.

The system 400 further includes an eccentric positioning element 403 fixed to the catheter body 401 adjacent and proximal the cutting head assembly 402. In some example embodiments, the eccentric positioning element 403 is an inflatable balloon position opposite the opening in the cutting head assembly 402. As such, when the balloon is inflated, the opening of the cutting head assembly 402 is pressed against a vessel wall.

Also shown in FIG. 2 are a guide wire 404 to facilitate insertion of the catheter body 401 and cutting head assembly 402 within a vessel, a motor 405 for rotating the cutting element of the cutting head assembly 402, and an Endoflator^{®} for use in inflation and deflation of the eccentric positioning element 403.

The system 400 of FIG. 2 and the embodiments that follow may be particularly suited to target collagenous lesions in the deep venous system. The cutting head assembly 402 may, for example, be between 1 and 3 cm, and may be made from a rigid material such as stainless steel (an extension of a laser-cut hypotube catheter body but uncut), nitinol, or a rigid thermoplastic like HDPE. The catheter body 401 may be reinforced with a laser-cut hypotube to achieve a balance of flexibility and torque response, which is desired for precise maneuvering within the deep venous system. As will be explained in the embodiments that follow, the interior of the catheter body 401 may be configured to accommodate three lumens. The first lumen serves as an aspiration pathway for transmitting negative pressure from a vacuum pump located in the console to the cutting head, facilitating the effective removal of macerated lesion pieces. The second lumen is dedicated to balloon inflation, providing the means for inflating the eccentric positioning balloon. A third lumen is dedicated for the guide wire.

In some embodiments, the cutting head assembly 402 may include two non-concentric shafts. The inner shaft may be a rotating cutting blade designed to macerate collagenous lesions while also serving as a material transport lumen on its interior. The outer shaft serves as a protective cage that may, for example, be between 1 and 3 cm in length, with an opening approximately 0.5 cm shorter than the cutting head length and a width of approximately 45 to 90 degrees measured from its center axis. It is emphasized here, however, that the inventive concepts are not limited to these dimensional examples. This design allows for directed lesion maceration while also providing protection for the blade beyond the treatment area. In the meantime, the eccentric positioning element 403 plays a role in providing targeted radial force on the cutting blade assembly 402. This radial force serves to push the blade into the lesion, thereby enhancing the ingress of lesion material into the cutting head assembly 402, optimizing the amount of lesion that can be effectively removed. Additionally, in some embodiments, the entire cutting head assembly 402 is able to reposition, allowing for a larger treatment area before needing to deflate the eccentric positioning balloon, rotate the catheter, and reinflate.

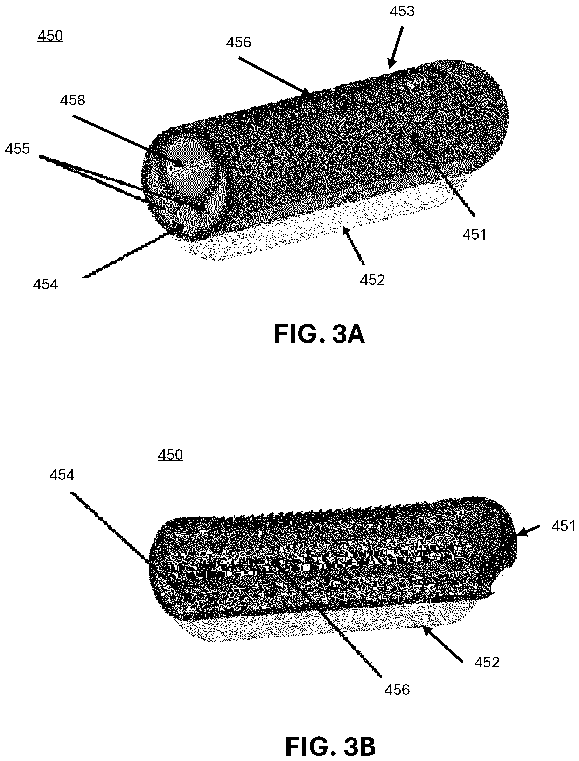

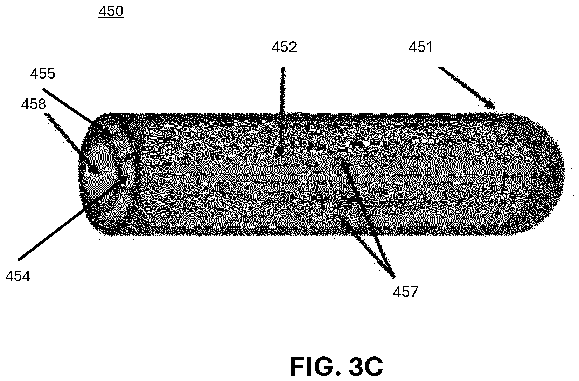

FIGS. 3A through 3C are schematic and cut-away views of a cutting head assembly according to some embodiments of the inventive concepts.

Referring collectively to FIGS. 3A through 3C, the cutting head assembly of this example includes a protective cage 451. As illustrated, the protective cage 451 is a hollow shaft that is closed at a distal end (except for a guide wire opening) and open at a proximal end. In addition, the protective cage 451 has an opening 453 formed in a side surface thereof. In this example of this embodiment, the opening is elongated in an axial direction.

Within the protective cage 451 is a cutting element 456. The cutting element 456 of this example is a hollow shaft that is non-concentric with the protective cage. The cutting element 456 may be closed at a distal end and open at a proximal end. In addition, the cutting element 456 may have opening formed in a side surface thereof. In the example of this embodiment, the opening is elongated in an axial direction. In operation, upon rotation of the cutting element 456, each time an elongate edge of the opening in the cutting element 456 passes an opposing edge of the opening in the protective cage 451, the two edges cooperate to form a scissoring action that macerates lesions of a vessel wall. One or both edges may be serrated to enhance the scissoring effect. As explained previously, rotation of the cutting element 456 may be achieved by a motor configured to rotate a drive shaft extending within the catheter body and coupled to the cutting element 456.

In the example of the present embodiment, the interior of the cutting element 456 forms an aspiration cavity 458 where lesion pieces macerated by the cutting element 456 are gathered. As explained previously, the aspiration cavity 458 may be in fluid communication with a vacuum pump through a material transport lumen in the catheter body. In alternative embodiments, the material transport lumen in the catheter may be configured as an Archimedes screw in fluid communication with the cavity 458. In that case, a motor at the console is configured to turn the screw for material transport instead of a vacuum pump at the console.

Also contained within the protective cage 451 is a guide wire lumen 454 for insertion therethrough of a guide wire. Inflation lumens 455 are defined within the protective cage 451 on opposite sides of the guide wire lumen 454. The inflation lumens 455 are in fluid communication with an eccentric positioning balloon 452 through balloon inflation ports 457 formed in the protective cage 451. Although two inflations lumens 455 are illustrated in the current embodiment, in alternative embodiments there may be a single inflation lumen or more than two inflation lumens.

In some embodiments of the inventive concepts, as in FIGS. 3A through 3C, the eccentric positioning balloon 452 is attached to the protective cage 451 of the cutting head assembly 450. In other embodiments, the eccentric positioning balloon is attached to the catheter body adjacent the cutting head assembly, and inflation ports are formed in the catheter body. In that case, the balloon 452, the inflation lumens 455 and balloon inflation ports 457 of FIGS. 3A through 3C may be omitted.

Regardless of whether the balloon 452 is attached to the cutting head assembly 450 or the catheter body, the balloon 452 when inflated provides a targeted radial force on the cutting blade assembly 450. This radial force serves to push the blade 456 of the cutting blade assembly 450 in a direction towards a vessel wall and thus into a lesion on the vessel wall. The ingress of lesion material into the opening 453 of the protective cage 451 is thereby enhanced, optimizing the amount of lesion that can be effectively removed.

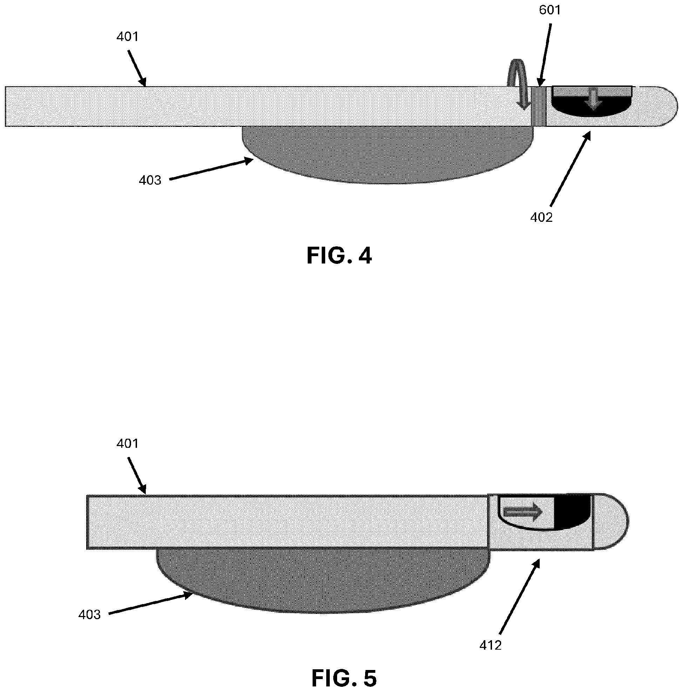

FIG. 4 is a schematic view for reference in describing another embodiment of the inventive concepts.

The configuration of the embodiment of FIG. 4 is similar to that of FIG. 2, except that the cutting head assembly 402 is rotatable at its interface 601 with the catheter 401. As such, the cutting head assembly 402 is independently rotatable about the longitudinal axis relative to the eccentric positioning balloon 403, thus increasing the amount of material that can be removed before deflating the balloon 403 and repositioning of the cutting assembly 402 within the vessel. In some examples, this embodiment is realized by the use of a separate drive shaft within the catheter body 401 that is coupled to the protective cage of the cutting head assembly 402.

FIG. 5 is a schematic view for reference in describing another embodiment of the inventive concepts.

The configuration of the embodiment of FIG. 5 is similar to that of FIG. 2, except that the embodiment of FIG. 5 employs a translating blade 412 rather than a rotating one to macerate a lesion. The translating blade 412 in cooperation with a radial edge of the opening in the protective cage is configured to macerate lesions of the vessel wall. In some examples, this embodiment is realized by the use of a cam shaft in the catheter body 401 to convert the rotational energy of a drive shaft to reciprocating energy to drive the translating blade 412.

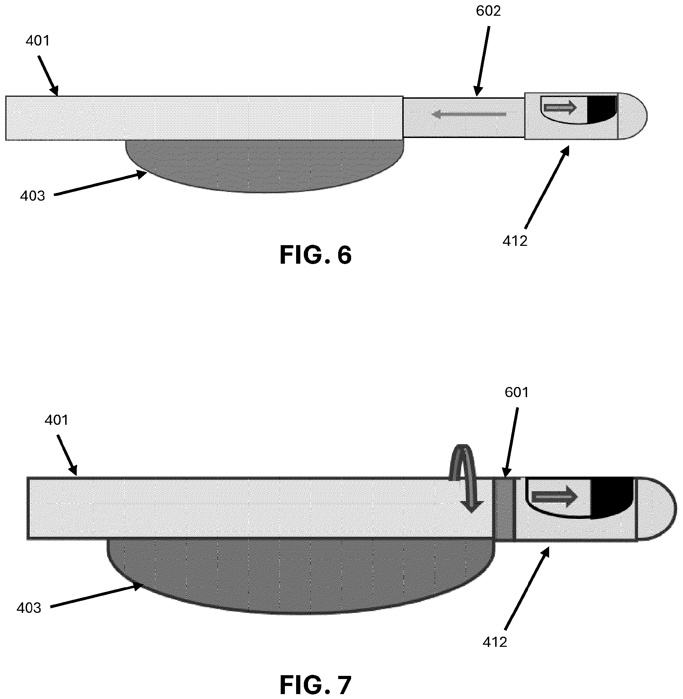

FIG. 6 is a schematic view for reference in describing another embodiment of the inventive concepts.

The configuration of the embodiment of FIG. 6 is similar to that of FIG. 5, except that the cutting head assembly 412 is configured to reciprocate longitudinally at the interface with the catheter 401. As such, the cutting head assembly 402 is independently movable along the longitudinal axis relative to the eccentric positioning balloon 403, thus increasing the amount of material that can be removed before deflating the balloon 403 and repositioning of the cutting assembly 402 within the vessel. In some examples, this embodiment is realized by the use of a separate reciprocating drive shaft 602 within the catheter body 401 that is coupled to the protective cage of the cutting head assembly 402.

FIG. 7 is a schematic view for reference in describing another embodiment of the inventive concepts.

The configuration of FIG. 7 is similar to that of FIG. 4, except that the embodiment of FIG. 7 employs a translating blade 412 rather than a rotating one to macerate a lesion. As such, the translating cutting head assembly 402 is independently rotatable about the longitudinal axis relative to the eccentric positioning balloon 403, thus increasing the amount of material that can be removed before deflating the balloon 403 and repositioning of the translating cutting assembly 412 within the vessel.

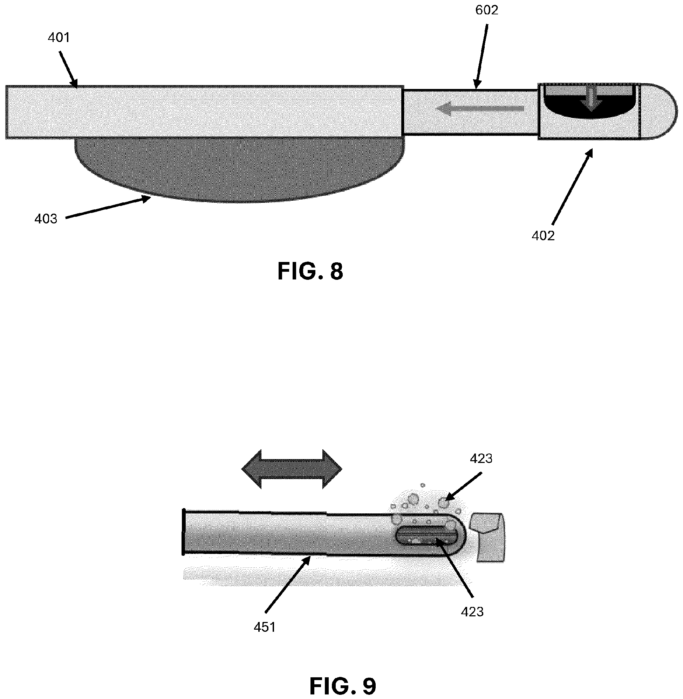

FIG. 8 is a schematic view for reference in describing another embodiment of the inventive concepts.

The configuration of the embodiment of FIG. 8 is similar to that of FIG. 6, except that the cutting head assembly 402 adopts a rotating blade rather than a translating blade. As such, the rotational cutting head assembly 402 is independently movable along the longitudinal axis relative to the eccentric positioning balloon 403, thus increasing the amount of material that can be removed before deflating the balloon 403 and repositioning of the cutting assembly 402 within the vessel.

FIG. 9 is a schematic view for reference in describing another embodiment of the inventive concepts.

The cutting action of the cutting head assembly is not limited to the scissoring action obtained between a blade (rotational or translating) and the edge in the opening of a protective cage as in the embodiments described above. FIG. 9 is a schematic view illustrating an example in which a radio frequency (RF) cutter 423 is used instead of a mechanical blade to macerate lesion material 423. The RF cutter 423 may, for example, be positioned at the opening of the protective cage 451. The assembly including the RF cutter 423 can be configured rotate as in the embodiments of FIGS. 4 and 7, and/or reciprocate as in the embodiments of FIG. 6 and 8. This embodiment would include an RF generator at the console to power the RF cutter 423, and electrical leads extending through the catheter body 401 between the RF generator and the RF cutter 423.

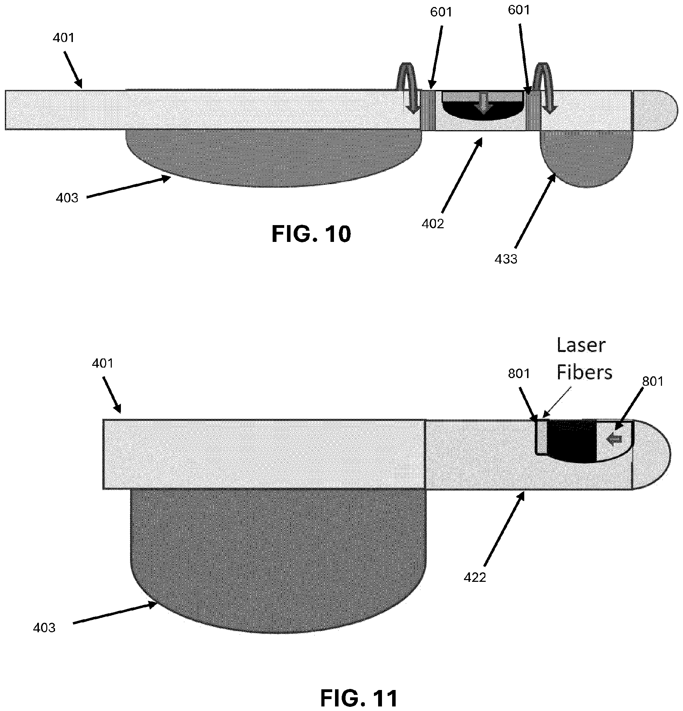

FIG. 10 is a schematic view for reference in describing another embodiment of the inventive concepts.

Referring to FIG. 10, this embodiment incorporates a proximal balloon 403 as well as a distal balloon 433 on opposite sides of the cutting head assembly 402. Also, in the illustrated embodiment, the cutting head assembly 402 is rotatable about the longitudinal axis independently of the balloons 403 and 433. Separately, this configuration may enable the application of vacuum pressure during the therapy to draw the lesion into the cutting mouth effectively.

FIG. 11 is a schematic view for reference in describing another embodiment of the inventive concepts.

Referring to FIG. 11, the cutting head assembly of this embodiment utilizes a laser-based cutter 801 instead of a mechanical blade. This laser-based cutter is configured in a similar translational configuration to the embodiment of FIG. 5, with the distinction that the cutting element (the laser) does not translate, while the translating piece pushes the lesion toward the cutter. This embodiment would include a laser source at the console, and optical fibers extending through the catheter body 401 for guiding laser light from the laser source to the laser-based cutter 801. The laser source may be a gas laser or a solid-state laser capable of ultraviolet (UV) light generation, for example, having a wavelength in a range of about 126nm to about 360nm, or more specifically in a range of about 300nm to about 360nm (e.g., a 308nm excimer laser).

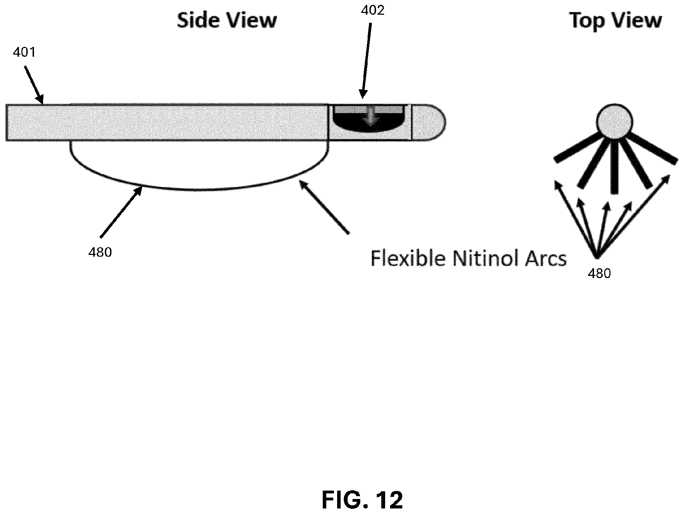

FIG. 12 is a schematic view for reference in describing another embodiment of the inventive concepts.

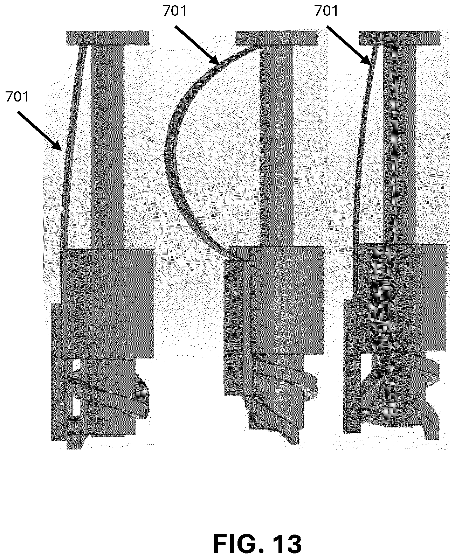

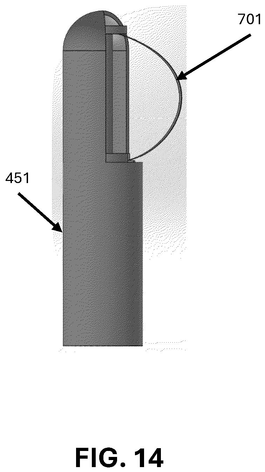

The inventive concepts are not limited to the use of a balloon(s) as the eccentric positioning element. Referring to FIG. 12, another alternative to is use one or more flexible nitinol arcs as an eccentric positioning element. In this embodiment, nitinol filaments extending partially through the catheter body 401 which includes elongate recesses (not shown) adjacent the cutting head assembly 401. A compressive force applied to the nitinol wires from a proximal end of the catheter body causes the nitinol filaments to arc beyond the recesses, thus providing the radial force on the cutter 402 instead of using a balloon. This embodiment would not include inflation lumens on the interior of the catheter or use an inflation device on the handle/console. In example embodiments, the flexible nitinol arcs 480, which may be a few centimeters in length, are distally attached to the proximal end of the cutting head assembly 402. The proximal ends of the arcs 480 may be attached to a sliding mechanism (not shown) within the catheter body 401. In it default state, the sliding mechanism is positioned proximally, relaxing the arcs and laying them flat. When the sliding mechanism is advanced forwarded (e.g., by sliding or rotating a mechanism on a handle of the device at the proximal end of the catheter 401), the proximal ends of the arcs 408 are pushed forward. This causes the arcs 408 to flex into an arc shape as shown in FIG. 12. FIGS. 13 and 14 are schematic views for reference in describing another embodiment of the inventive concepts.

The embodiment of FIGS. 13 and 14 is similar to previous embodiments, except that a blade 701 is configured is to extend outside the cutting mouth or opening of the protective cage 451 (see FIG. 14). The blade 701 follows a ramp, which compresses and relaxes the blade 701, acting as a cam to transform the rotational motion of the shaft into linear motion for the blade to extend out of the cutting mouth. The distal end of the blade remains fixed while the proximal end follows a helical path. Initially relaxed within the protective cage 451, as the blade rotates and nears the opening, the proximal end follows the ramp distally, compressing and causing the blade to bow outward and protrude out of the cutting mouth until it reaches the center. Subsequently, the proximal end follows the ramp proximally, relaxing the blade and allowing it to recede back into the protective cage 451. When actuated, the blade 701 continues to rotate about its axis as the blade 701 is repeatedly compressed and relaxed. While a single blade 701 is illustrated in FIGS. 13 and 14, it will be understood that the cutting mechanism can be equipped with two or more blades.

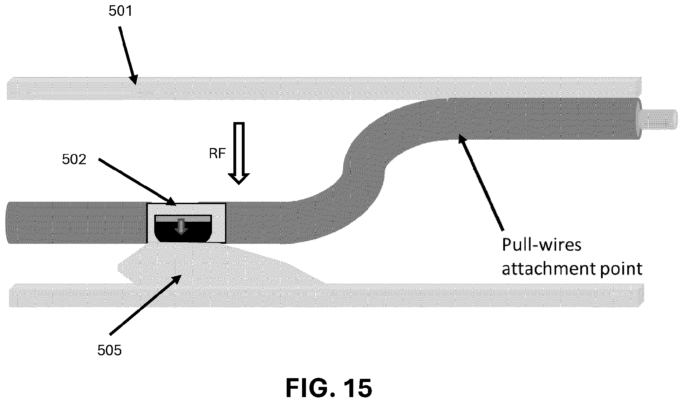

FIG. 15 is a schematic view for reference in describing another embodiment of the inventive concepts.

As described previously in connection with the embodiment of FIG. 12, the inventive concepts are not limited to the use of a balloon(s) as the eccentric positioning element. Referring to FIG. 15, another alternative to use pull wires and variable stiffness sections of the elongate catheter to provide the radial force RF on cutting head 502 to macerate the lesions 505 on the vessel wall.

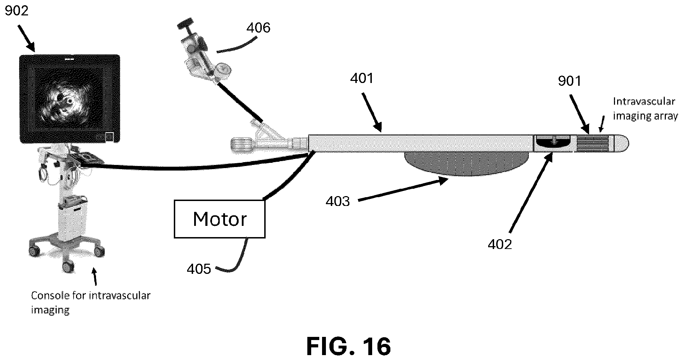

FIG. 16 is a schematic view for reference in describing another embodiment of the inventive concepts.

The embodiment of FIG. 16 is similar to the embodiment of FIG. 2, except that the embodiment of FIG. 16 includes intravascular imaging elements 901 added at the distal end of the catheter adjacent the cutting head assembly 402. These imaging elements 901 are connected through the catheter to an imaging console 902. The embodiment offers the advantage of the lesion maceration process being guided/monitored via intravascular imaging (e.g. intravascular ultrasound (IVUS) or optical coherence tomography (OCT)) of the lesion.

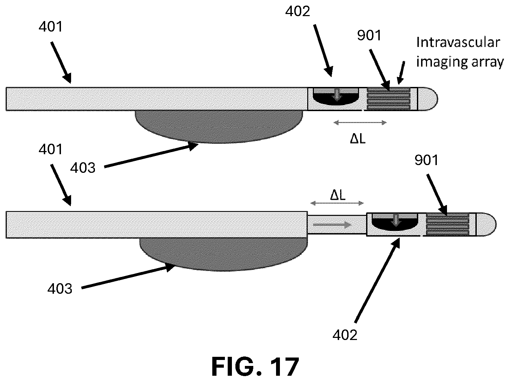

FIG. 17 is a schematic view for reference in describing another embodiment of the inventive concepts.

The embodiment of FIG. 17 is similar to the embodiment of FIG. 16, except that in the embodiment of FIG. 17, the cutting head assembly 402 together with the imaging elements 901 can be translated longitudinally relative to the catheter 401 and balloon 403. In an example embodiment, the amount of translation is the same the length difference (ΔL) between the imaging elements 901 and the morcellator of the cutting head assembly 402.

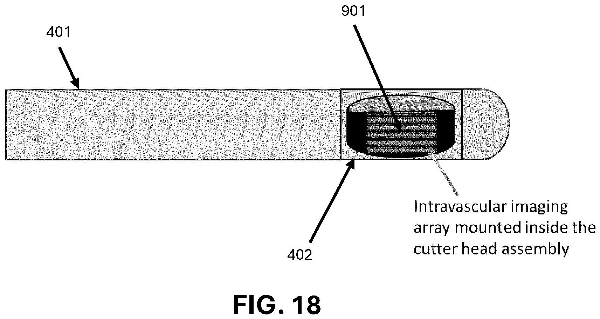

FIG. 18 is a schematic view for reference in describing another embodiment of the inventive concepts.

The embodiment of FIG. 18 is similar to the embodiment of FIG. 16, except that in the embodiment of FIG. 18 the imaging elements 901 are located inside the cutting head assembly 402. In this embodiment, the frequency of the image generation may be set such the image is captured when the cutting blade of the cutting head assembly 402 is not in the way to allow constant visualization.

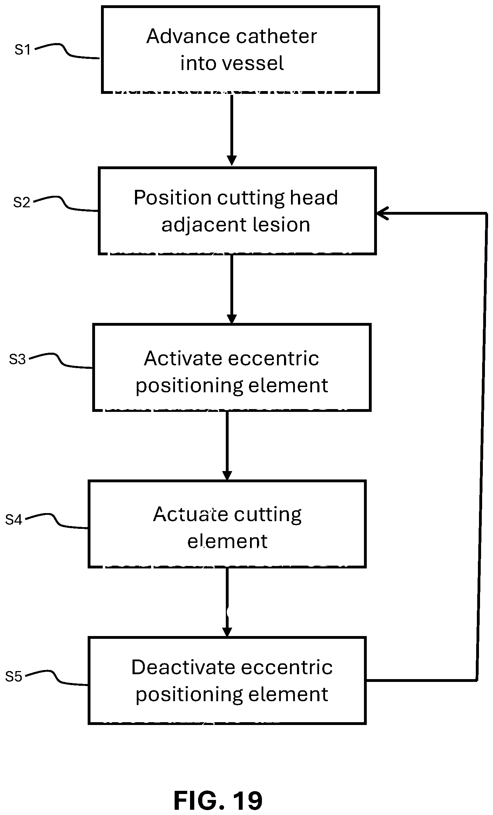

FIG. 19 is a flowchart for reference in describing a method of macerating vasculature lesions according to an embodiment of the inventive concepts.

Referring to FIG. 19, as S1, a catheter is advanced into a vessel. The catheter includes a cutting head assembly with a cutting element facing outwardly towards a vessel wall from a longitudinal axis of the catheter as in any of the embodiments described above.

Next, at S2, the cutting head assembly is position adjacent to a lesion of the vessel wall with the cutting element facing the lesion.

Next, at S3, an eccentric positioning element is activated to press the cutting element against the lesion. In some embodiments as described above, activating the eccentric positioning element includes inflating one or more balloon attached to the catheter.

Next, at S4, the cutting element is actuated to macerate the lesion. This may include rotating the cutting element and/or reciprocating the cutting element while the eccentric positioning element is activated to press the cutting element against the lesion.

Next, at S5, the eccentric positioning element is deactivated. In some embodiments as described above, deactivating the eccentric positioning element includes deflating one or more balloons attached to the catheter. The catheter is then extracted from the vessel, or as in the case of FIG. 19, the process returns to S2 to reposition cutting head assembly, reactivate the eccentric positioning element, and continue maceration of the lesion within the vessel.

In alternative embodiments, the cutting head assembly is repositioned without deactivating the eccentric position element. In the context of FIG. 19, this would mean that the process proceeds from S4 to S2 (i.e., omits S5). This can occur when the balloon, for example, is a compliant or semi-compliant balloon.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. While representative embodiments are disclosed herein, one of ordinary skill in the art will appreciate that many variations that are in accordance with the present teachings are possible and remain within the scope of the appended claim set. The invention therefore is not to be restricted except within the scope of the appended claims.

### Additional embodiments

Embodiment 1. A lesion maceration catheter, comprising:
an elongate catheter body;
a cutting head assembly disposed at a distal end of the catheter body, the cutting head assembly comprising a protective cage with an opening in a side surface of the protective cage, and a cutting element within the protective cage that is exposed through the opening; and
an eccentric positioning element configured to press the opening of the cutting head assembly against a lesion on a vessel wall, thereby pushing the lesion into the opening in the cutting head assembly.

Embodiment 2. The lesion maceration catheter of Embodiment 1, wherein the eccentric positioning element is positioned proximal to the cutting head.

Embodiment 3. The lesion maceration catheter of Embodiment 2, wherein the eccentric positioning element is an inflatable balloon.

Embodiment 4. The lesion maceration catheter of Embodiment 1, wherein the cutting element comprises a cutting blade.

Embodiment 5. The lesion maceration catheter of Embodiment 4, wherein the cutting blade in cooperation with an edge of the opening in the protective cage is configured to macerate lesions of the vessel wall.

Embodiment 6. The lesion maceration catheter of Embodiment 5, wherein the cutting blade and/or the edge of the opening in the protective cage is serrated.

Embodiment 7. The lesion maceration catheter of Embodiment 4, wherein the cutting blade is a rotating cutting blade.

Embodiment 8. The lesion maceration catheter of Embodiment 4, wherein the cutting blade is a translating cutting blade.

Embodiment 9. The catheter system of Embodiment 1, wherein the elongate catheter body comprises a material transport lumen for removal of lesion pieces macerated by the cutting head assembly, the material transport lumen in fluid communication with an interior of the cutting head assembly.

Embodiment 10. The lesion maceration catheter of Embodiment 1, further comprising a rotating cutting blade within the catheter and configured to further cut the lesion pieces macerated by the cutting head assembly.

Embodiment 11. A lesion maceration catheter, comprising:
an elongate catheter body;
a cutting head assembly disposed at a distal end of the catheter body, the cutting head assembly comprising a protective cage with an opening in a side surface of the protective cage, and a rotational or translating cutting blade within the protective cage and exposed through the opening; and
an inflatable balloon mounted to the elongate catheter body, the inflatable balloon configured when inflated to press the opening of the protective cage against a lesion on a vessel wall, thereby pushing the lesion into the opening in the cutting head assembly;
an inflation lumen within the elongate catheter body in fluid communication with the inflatable balloon; and
a material transport lumen within the elongate catheter body in fluid communication with an interior of the cutting head assembly.

Embodiment 12. The lesion maceration catheter of Embodiment 11, wherein the cutting head assembly is independently movable in a longitudinal direction relative to the catheter body, and/or independently rotatable about the longitudinal axis relative to the catheter body.

Embodiment 13. The lesion maceration catheter of Embodiment 11, further comprising an intravascular imaging device at the distal end of the catheter body.

Embodiment 14. The lesion maceration catheter of Embodiment 11, further comprising:
a pump at a proximal end of the elongate catheter body in fluid communication with the inflation lumen;
a pump at the proximal end of the elongate catheter in fluid communication with the material transport lumen; and
a drive motor at the proximal end of the elongate catheter operatively connected to the rotational or translating cutting blade by at least one drive shaft extending through the catheter body.

Embodiment 15. A method for macerating a vasculature lesion, comprising:
advancing a catheter into a vessel, the catheter comprising a cutting head assembly with a cutting element facing outwardly towards a vessel wall from a longitudinal axis of the catheter;
positioning the cutting head assembly adjacent to a lesion of the vessel wall;
activating an eccentric positioning element to press the cutting element against the lesion; and
actuating the cutting element to macerate the lesion.

## Claims

1. A lesion maceration catheter, comprising:
an elongate catheter body;
a cutting head assembly disposed at a distal end of the catheter body, the cutting head assembly comprising a protective cage with an opening in a side surface of the protective cage, and a cutting element within the protective cage that is exposed through the opening; and
an eccentric positioning element configured to press the opening of the cutting head assembly against a lesion on a vessel wall, thereby pushing the lesion into the opening in the cutting head assembly.

2. The lesion maceration catheter of claim 1, wherein the eccentric positioning element is positioned proximal to the cutting head.

3. The lesion maceration catheter of claim 2, wherein the eccentric positioning element is an inflatable balloon.

4. The lesion maceration catheter of any one of the claims 1 to 3, wherein the cutting element comprises a cutting blade.

5. The lesion maceration catheter of claim 4, wherein the cutting blade in cooperation with an edge of the opening in the protective cage is configured to macerate lesions of the vessel wall.

6. The lesion maceration catheter of claim 5, wherein the cutting blade and/or the edge of the opening in the protective cage is serrated.

7. The lesion maceration catheter of claim 4, wherein the cutting blade is a rotating cutting blade.

8. The lesion maceration catheter of claim 4, wherein the cutting blade is a translating cutting blade.

9. The lesion maceration catheter of any one of the claims 1 to 8, wherein the elongate catheter body comprises a material transport lumen for removal of lesion pieces macerated by the cutting head assembly, the material transport lumen in fluid communication with an interior of the cutting head assembly.

10. The lesion maceration catheter of any one of the claims 1 to 9, further comprising a rotating cutting blade within the catheter and configured to further cut the lesion pieces macerated by the cutting head assembly.

11. The lesion maceration catheter of claim 3,
wherein the cutting element comprises a rotational or translating cutting blade, and wherein the inflatable balloon is mounted to the elongate catheter body, the lesion maceration catheter comprising:
an inflation lumen within the elongate catheter body in fluid communication with the inflatable balloon; and
a material transport lumen within the elongate catheter body in fluid communication with an interior of the cutting head assembly.

12. The lesion maceration catheter of claim 11, wherein the cutting head assembly is independently movable in a longitudinal direction relative to the catheter body, and/or independently rotatable about the longitudinal axis relative to the catheter body.

13. The lesion maceration catheter of claim 11 or 12, further comprising an intravascular imaging device at the distal end of the catheter body.

14. The lesion maceration catheter of any one of claims 11 to 13, further comprising:
a pump at a proximal end of the elongate catheter body in fluid communication with the inflation lumen;
a pump at the proximal end of the elongate catheter in fluid communication with the material transport lumen; and
a drive motor at the proximal end of the elongate catheter operatively connected to the rotational or translating cutting blade by at least one drive shaft extending through the catheter body.

15. A method for macerating a vasculature lesion, comprising:
advancing a catheter into a vessel, the catheter comprising a cutting head assembly with a cutting element facing outwardly towards a vessel wall from a longitudinal axis of the catheter;
positioning the cutting head assembly adjacent to a lesion of the vessel wall;
activating an eccentric positioning element to press the cutting element against the lesion; and
actuating the cutting element to macerate the lesion.
